# EUROPÄISCHE PATENTSCHRIFT

(11) **EP 2 524 710 B1**
(45) Veröffentlichungstag und Bekanntmachung des Hinweises auf die Patenterteilung: **19.12.2018**
(21) Anmeldenummer: 11075088.2
(22) Anmeldetag: 16.05.2011
(51) Int. Cl.: A61M 1/10, F16L 11/11

(54) **Kanüle zur Bildung eines Fliesskanals für Körperflüssigkeiten**
Cannula for establishing a flow channel for bodily fluids
Canule pour la formation d'un canal d'écoulement pour liquides corporels

(43) Veröffentlichungstag der Anmeldung: 21.11.2012
(60) Teilanmeldung: 18205776.0
(73) Patentinhaber: Berlin Heart GmbH, 12247 Berlin (DE)
(72) Erfinder: Göllner, Manfred, 13086 Berlin (DE); de Souza, Marvin, 12209 Berlin (DE); Schellhase, Joachim, 12103 Berlin (DE); Samlenski, Uwe, 12169 Berlin (DE)
(74) Vertreter: Pfenning, Meinig & Partner mbB

(56) Entgegenhaltungen:
- EP-A2- 1 496 301
- DE-A1- 2 913 510
- DE-U1- 20 307 410
- US-A- 3 908 704
- US-A- 4 334 327
- US-A1- 2004 059 178
- US-A1- 2007 066 943
- US-A1- 2007 197 855

## Beschreibung

Die Erfindung liegt auf dem Gebiet der Mechanik und ist insbesondere im Bereich der Medizintechnik mit Vorteil anwendbar. Sie bezieht sich auf eine Kanüle zur Bildung eines Fließkanals für körpereigene Flüssigkeiten.

Unter einer Kanüle wird in diesem Zusammenhang ein Leitungselement, d.h. ein Hohlkörper, verstanden, der meistens zylindrisch ausgebildet ist und aus einem biokompatiblen Werkstoff besteht, so dass er beispielsweise bei Operationen oder auch allgemein bei der Behandlung von Patienten in körpereigenes Gewebe eingebettet werden kann. Kanülen können mehr oder weniger starr oder auch in Grenzen biegsam ausgeführt sein.

Einen Sonderfall derartiger Kanülen stellen die sogenannten Grafts dar, die üblicherweise im Bereich des Ersatzes für Blutgefäße verwendet werden und die durch Annähen z.B. an ein Blutgefäß direkt angekoppelt werden können. Solche Grafts sind an sich bekannt und bestehen üblicherweise aus einem sehr flexiblen, teilweise für Blut durchlässigen Gewebewerkstoff, der erst durch die Tränkung mit Blut, das sogenannte Glotting, im Verlauf des Gebrauchs abgedichtet wird. Derartige Grafts werden üblicherweise entweder direkt oder mittels eines sogenannten Nahtrings an Blutgefäßen oder Teilen eines Herzens befestigt.

Ein grundsätzliches Problem bei der Verwendung derartiger Grafts ist außer der anfänglichen Undichtigkeit die notwendige Flexibilität, die auch eine Verformung während einer Operation oder eine Kürzung regelmäßig möglich macht. Je nach dem Winkel, in dem ein Graft an ein körpereigenes Gefäß angekoppelt wird, ist auch eine Biegung des Grafts zum Anschluss beispielsweise an einen Filter oder eine Herzpumpe notwendig. In diesem Zusammenhang ist oft auch eine Kürzungsmöglichkeit wichtig, so dass die im Leitungsverlauf dem Graft nachfolgenden Teile (Fortsetzungskanülen aus PUR oder Silikon) in vorhersehbarer und zuverlässiger Weise im Körper oder am Körper verlegt werden können. Beispielsweise werden oft Leitungsstücke mit einer veloursartig aufgerauten Oberfläche zum Durchtritt durch die Haut des Patienten versehen, damit dort durch ein Anwachsen an das Leitungsstück ein besonders effektiver Wundverschluss gewährleistet werden kann. Wenn durch eine flexible Formgebung und Kürzung des Grafts bzw. der erfindungsgemäßen Kanüle bewirkt werden kann, dass nur noch eine geringe Varianz bei der Verschiebung der übrigen Teile der Leitung benötigt wird, so wird hierdurch ein großer Vorteil bei der Handhabung und im Materialverbrauch erreicht.
Grundsätzlich ist eine Kanülenanordnung mit einem flexiblen Wellschlauch aus der US 2004/059178 A1 bekannt.
Die US 2007/066943 A1 offenbart eine Kanüle in Form eines als Wellschlauch gestalteten Grafts.
Aus der US 3 908 704 A ist ein Herstellungsverfahren für einen Wellschlauch bekannt.
Der vorliegenden Erfindung liegt damit die Aufgabe zugrunde, eine Kanüle zu schaffen, die möglichst einfach und mit geringem konstruktivem und materiellem Aufwand herstellbar, gut verwendbar und verarbeitbar und zuverlässig mit anderen Teilen einer Leitungsführung verbindbar ist. Sie sollte vorteilhaft zudem anfangsdicht sein, d.h. nicht zur Abdichtung einer Tränkung bedürfen, und eine Eigenstabilität aufweisen, die ihr eine Formstabilität auch bei einem gewissen Unterdruck verleiht.
Die Aufgabe wird gemäß der Erfindung mit den Merkmalen des Patentanspruchs 1 gelöst.
Dabei ist die erfindungsgemäße Kanüle typischerweise ein Graft zur Bildung eines Fließkanals für Körperflüssigkeiten, insbesondere für Blut, mit einem ersten Ende, das zur mittelbaren oder unmittelbaren Verbindung mit einem Körperorgan, beispielsweise einem Herzen, oder einem Körperflüssigkeit führenden körpereigenen Hohlraum, beispielsweise einem Blutgefäß, ausgebildet ist, und mit einem zweiten Ende, das zur Verbindung mit einem Leitungselement, beispielsweise einer weiteren Kanüle, oder einem Funktionselement der Flüssigkeitsförderung, beispielsweise einer Pumpe, ausgebildet ist.
Gemäß der Erfindung ist vorgesehen, dass die Kanüle wenigstens zwischen ihren Enden aus einem homogenen, von Anfang an flüssigkeitsdichten Material besteht, das elastisch und/oder plastisch verformbar ist, dass die Kanüle durch Spritzgießen aus Silikon, einem Silikonelastomer oder einem Polyurethan, hergestellt ist und dass die Wanddicke und/oder der Durchmesser der Kanüle im Längsschnitt zwischen dem ersten und dem zweiten Ende im Profil zwei oder mehr Maxima und Minima aufweist.
Dabei bewirken die Maxima der Wanddicke eine Versteifung der Kanüle insoweit, als der Innendurchmesser der Kanüle bei einer Biegung näherungsweise erhalten bleibt und die Kanüle nicht leicht zusammendrückbar ist.
Die Minima der Wanddicke erlauben eine Biegung der Kanüle, wobei der Innendurchmesser jeweils näherungsweise erhalten bleibt. Die Kanüle ist auch in Grenzen unterdruckstabil.
Bei der Ausbildung der Kanüle mit Maxima und Minima des Durchmessers entlang der Kanülenachse wird nach Art eines Wellschlauches ebenfalls eine Stabilität des Innendurchmessers, anders ausgedrückt eine Stabilität der Form des inneren Hohlraums der Kanüle, sichergestellt, während gleichzeitig eine Biegung der Kanüle ermöglicht wird.

Das Konzept der Erfindung kann alternativ auch dadurch ausgedrückt werden, dass das Profil der Kanüle im Längsschnitt entlang ihrer Längsachse wiederkehrende, insbesondere äquidistante Durchmesserveränderungen und/oder Wandverdickungen aufweist.

Vorteilhaft kann die Erfindung dadurch ausgestaltet sein, dass die wiederkehrenden Wandverdickungen kreisringförmig umlaufend ausgebildet sind. Diese Wandverdickungen können jedoch alternativ auch spiralförmig umlaufend ausgebildet sein, ebenso wie entsprechende Durchmesserveränderungen in Form von wellenförmigen Zu- und Abnahmen des Durchmessers.

Ein besonderer Vorteil wird dabei dann erreicht, wenn der Innendurchmesser der Kanüle im Bereich von Wandverdickungen entlang der Längsachse der Kanüle konstant ist. Hierdurch werden sogenannte Totwassergebiete vermieden, und die Strömung der Flüssigkeit, insbesondere des Blutes, im Inneren der Kanüle wird von strömungsbehindernden Durchmesseränderungen nicht gehemmt. Dies kann bei einer wellschlauchförmigen Kanüle auch beispielsweise dadurch erreicht werden, dass auf der Innenseite eine Verfüllung der Wandausbauchungen mit einem Material vorgenommen wird, das wesentlich leichter verformbar ist als das Material, aus dem die Kanüle besteht, beispielsweise einem Schaumstoff.

Besonders vorteilhaft besteht die Kanüle wenigstens teilweise aus einem Silikon bzw. Silikonelastomer.

Dabei kann ein Anschlussstück oder ein Flansch am ersten Ende der Kanüle zur Verbindung mit einem Blutgefäß oder einem Körperorgan zusammen mit den übrigen Teilen der Kanüle einstückig im Spritzgussverfahren hergestellt sein, oder ein solches Anschlussstück kann nach der Herstellung mit der Kanüle verklebt oder in anderer Weise verbunden werden. Dabei kann z.B. vorgesehen sein, dass die Verbindung zu der Kanüle derart ausgebildet ist, dass die Kanüle trotz Flüssigkeitsdichtigkeit noch gegenüber dem Anschlussstück um ihre Längsachse drehbar ist.
Eine vorteilhafte Ausgestaltung der Erfindung sieht außerdem vor, dass die Kanüle im entspannten Zustand eine Vorzugskrümmung aufweist. In diesem Fall kann die Kanüle vor der Befestigung im Patientenkörper oder auch danach, falls eine drehbare Verbindung besteht, in die richtige Position gebracht werden, so dass die vorhandene Krümmung der im Einzelfall benötigten Krümmung möglichst weitgehend entspricht. Es kann auch eine doppelt gegensinnig gekrümmte Form (S-Form) im entspannten Zustand vorgesehen sein.
Die Erfindung bezieht sich außer auf eine Kanüle auch auf einen Kanülenverbinder gemäß Anspruch 10 für eine oben beschriebene Kanüle, bei dem ein in die Kanüle einsteckbarer Stutzen und ein außen an dem Stutzen azimutal umlaufender Steg sowie ein den Steg einerseits und wenigstens einen ersten bezüglich des Außendurchmessers ein Maximum aufweisenden Längsbereich der Kanüle andererseits umgreifender Profilring vorgesehen sind. Der Profilring hält dabei den Steg des Stutzens des Kanülenverbinders und die Kanüle axial zusammen.
Der Profilring weist hierzu beispielsweise im Querschnitt (Radialschnitt) ein U-förmiges Profil mit zwei Schenkeln auf, die jeweils radial in Bezug auf die Kanüle nach innen weisen und die so weit voneinander beabstandet sind, dass sie den Steg an dem Stutzen einerseits und ein Wanddurchmessermaximum der Kanüle andererseits umgreifen.

Erfindungsgemäß ist der Profilring in zwei oder mehr Ringsegmente geteilt, die einzeln radial zusammengefügt und dabei auf die Kanüle und den Stutzen aufgesetzt werden können. Die verschiedenen Segmente/Sektoren des Profilrings werden vorteilhaft durch einen gemeinsamen, die Sektoren umgreifenden Haltering zusammengefasst und gehalten. Der Haltering kann entweder in axialer Richtung auf die Profilringsektoren aufschiebbar oder auch durch radiale Aufweitung auf diese aufschnappbar sein. Dazu kann der Haltering beispielsweise geschlitzt sein.
Der Haltering kann auch mit den Profilringsektoren, beispielsweise durch Kleben, fest verbunden sein. Er kann auch aus einem weichelastischen Material wie Silikon bestehen. Der Haltering kann ein oder mehrere Gelenke zwischen den Profilringsektoren aufweisen, mittels deren diese auf den Kanülenverbinder aufklappbar sind (in radialer Richtung). Die Profilringsektoren können auch durch einen umbördelbaren Schnappring aus weichelastischem Material gehalten werden - mit oder ohne einen Haltering.
Zudem kann zusätzlich zu dem Haltering auf dem Stutzen noch ein umbördelbarer Aufschnappring vorgesehen sein, der nach dem Aufsetzen der Profilringsektoren auf Kanüle und Stutzen sowie des Halterings auf den Profilring auf die Kombination von Profilring und Haltering bzw. über diese hinweg auf die Kanüle aufgeschnappt werden kann, um die Verbindung zusätzlich zu halten und zu schützen. Der Schnappring stellt damit ein mechanisches Halteelement dar, jedoch zusätzlich auch ein die Verbindungsstelle zwischen der Kanüle und einem weiteren Element überdeckendes Dichtelement.

Zur weiteren Verbesserung des Zusammenhalts kann auch ein auf die Kanüle aufschnappbarer Spannring, beispielsweise ein geschlitzter Ring aus Titan, vorgesehen sein, der in einem Bereich geringerer Wanddicke oder geringeren Außendurchmessers der Kanüle auf diese aufgeschnappt werden kann und der einen Innendurchmesser aufweist, der derart auf die Maße der Kanüle abgestimmt ist, dass der Spannring einen. radialen Druck auf die Kanüle in diesem Bereich ausübt. Der Spannring dient für einen aufschiebbaren Profilring als Axialanschlag. Vorteilhaft wird die Kanüle auf ein in diese eingeschobenes Leitungselement, beispielsweise einen Leitungsstutzen, zur Herstellung einer Flüssigkeitsdichtung aufgedrückt.

Im Folgenden wird die Erfindung anhand eines Ausführungsbeispiels in einer Zeichnung gezeigt und nachfolgend beschrieben. Dabei zeigt
- Fig. 1: eine Kanüle, die eine Aorta mit einer Pumpe verbindet und dabei die Haut eines Patienten durchdringt,
- Fig. 2: eine andere Ausgestaltung einer Kanüle zur Verbindung einer Aorta mit einer Pumpe, wobei die Kanüle aus verschiedenartigen Teilen zusammengesetzt ist,
- Fig. 3: eine Kanüle zwischen einer Aorta und einer implantierten Pumpe, die im Wesentlichen als ein durchgehender Graft ausgebildet ist,
- Fig. 4: eine erfindungsgemäße Kanüle mit einem Anschlussstück,
- Fig. 5: schematisch eine vorgekrümmte Kanüle,
- Fig. 6: eine erfindungsgemäße Kanüle mit spiralig umlaufenden und ringförmig umlaufenden Wandverdickungen in einer Außenansicht,
- Fig. 7: die Kanüle aus Fig. 6 in einem Längsschnitt,
- Fig. 8: eine Kanüle mit äquidistanten Wandverdickungen in einer Außenansicht,
- Fig. 9: die Kanüle aus Fig. 8 in einem Längsschnitt,
- Fig. 10: eine als Wellrohr aufgebaute Kanüle in einer Außenansicht,
- Fig. 11: die Kanüle aus Fig. 10 in einem Längsschnitt,
- Fig. 12: eine perspektivische Ansicht einer erfindungsgemäßen Kanüle mit einer Detailvergrößerung,
- Fig. 13: einen Längsschnitt durch einen Stutzen, der ein Verbindungsstück für eine Kanüle bilden kann,
- Fig. 14: den Stutzen aus Fig. 13 mit einer aufgeschobenen Kanüle,
- Fig. 15: zwei durch einen Haltering verbundene Profilringsektoren in einer perspektivischen Ansicht,
- Fig. 16: einen Längsschnitt, umfassend eine Kanüle, einen Verbindungsstutzen sowie einen aus zwei Profilringsektoren bestehenden Profilring und einen Haltering,
- Fig. 17: einen Längsschnitt wie in Fig. 16, wobei zusätzlich ein Schnappring über die Verbindungsstelle geschnappt ist, sowie
- Fig. 18: im Längsschnitt ein Kanülenende, einen Verbindungsstutzen, einen in wenigstens zwei Sektoren geteilten Profilring, einen Haltering und einen Schnappring in einer weiteren Ausgestaltung.

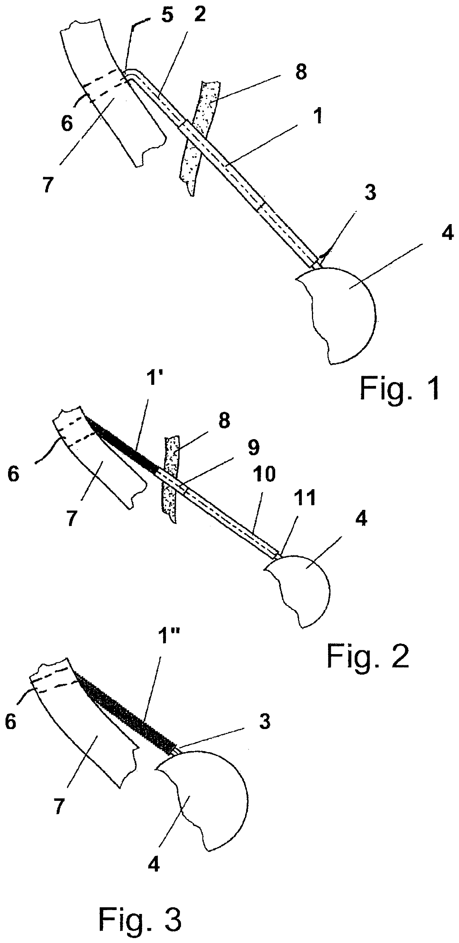

**Figur 1** zeigt eine Kanüle 1 mit einem vorderen Ende 2, einem hinteren Ende 3, das mit einer Blutpumpe 4 verbunden ist, sowie einem Kopf 5, der mit einer Öffnung 6 in einer Aorta 7 verbunden ist.

Durch die Öffnung 6 kann beispielsweise mittels einer Pumpe 4 über die Kanüle 1 Blut in die Aorta des Patienten eingeleitet werden.

Die Kanüle 1 weist verschiedene Abschnitte auf, von denen der mittlere Abschnitt die Bauchdecke 8 des Patienten durchdringt. In diesem Bereich kann die Kanüle 1 beispielsweise außen mit einem Veloursmaterial, beispielsweise aus Polyester, versehen sein.

Wenn die Kanüle 1, 2, 3 eine konstante Länge hat, wird es schwierig, im Vorhinein, beispielsweise vor einer Operation, abzuschätzen, wie groß die Entfernung zwischen der Öffnung 6 in der Aorta und der Bauchdecke 8 innerhalb des Patientenkörpers ist und an welcher Stelle entlang der Kanüle diese eine Velourschicht tragen muss, um das bessere Anwachsen der Haut an die Kanülenaußenseite zu ermöglichen.

Da die Kanüle als Silikonkanüle im Bereich des Kanülenkopfes 5 oft eine Kröpfung oder ein anderes Anschlussstück aufweist, kann sie an diesem Ende auch nicht gekürzt werden. Eine Kürzung muss deshalb im Bereich der Verbindung mit der Pumpe 4 stattfinden. Damit kann aber die Entfernung zwischen der Öffnung 6 in der Aorta 7 und der Hautschicht 8 nicht angepasst werden.

**Figur 2** zeigt eine Kanüle 1' in Form eines Grafts nach Art eines Wellenschlauches. Dieser ist auf der einen Seite mit der Aorta 7 im Bereich einer entsprechenden Öffnung 6 verbunden. Hierzu ist die Kanüle 1' / der Graft schräg angeschnitten. Es kann in diesem Bereich auch zusätzlich ein Anschlussstück, beispielsweise in Form eines Nahtrings, mit einem Anschlussstutzen vorgesehen sein.

Die Kanüle 1' ist mit einer Fortsetzungskanüle 9 fest und unlösbar verbunden, deren hinteres Ende 10 mit einer Pumpe 4 im Bereich eines Pumpenanschlusses 11 verbunden ist. Zwischen der Kanüle 1' und der Fortsetzungskanüle 9 ist ein Übergang vorgesehen, die innerhalb der Bauchdecke 8 im Inneren des Patientenkörpers liegt. Der Graft 1' kann üblicherweise durch Abschneiden gekürzt werden, so dass die Position der Fortsetzungskanüle 9 im Bereich der Durchquerung der Hautschicht 8 stabil gehalten werden kann.

Das Problem bei handelsüblichen Grafts ist, dass diese, wenn sie die erforderliche Flexibilität aufweisen, aus einem teils durchlässigen Gewebe bestehen, das erst durch Glotting, d.h. Bluttränkung, abgedichtet wird. Außerdem besteht das Risiko, einen solchen Graft, wenn er entsprechend biegsam ist, zu knicken, d.h., den Durchmesser der durchgehenden Öffnung zu verringern.

Der Einsatz eines erfindungsgemäßen Grafts im Rahmen des Bauteils 1' löst diese Probleme, indem ein anfangsdichter Werkstoff verwendet wird, beispielsweise Silikon oder Polyurethan, und dieser entsprechend der Erfindung als Wellschlauch oder mit wiederkehrenden Wandverdickungen gestaltet wird.

**Figur 3** zeigt einen entsprechend länger gestalteten Graft 1" der an seinem gefäßseitigen Ende mit der Aorta 7 im Bereich der Öffnung 6 verbunden und an seinem gegenüberliegenden Ende 3 derart verlängert ist, dass er bis zu der Pumpe 4 reicht. Die Kanüle / Der Graft 1" kann gemäß der Erfindung je nach den konkreten Bedürfnissen des Patienten in der Operationssituation gekürzt und gebogen werden.

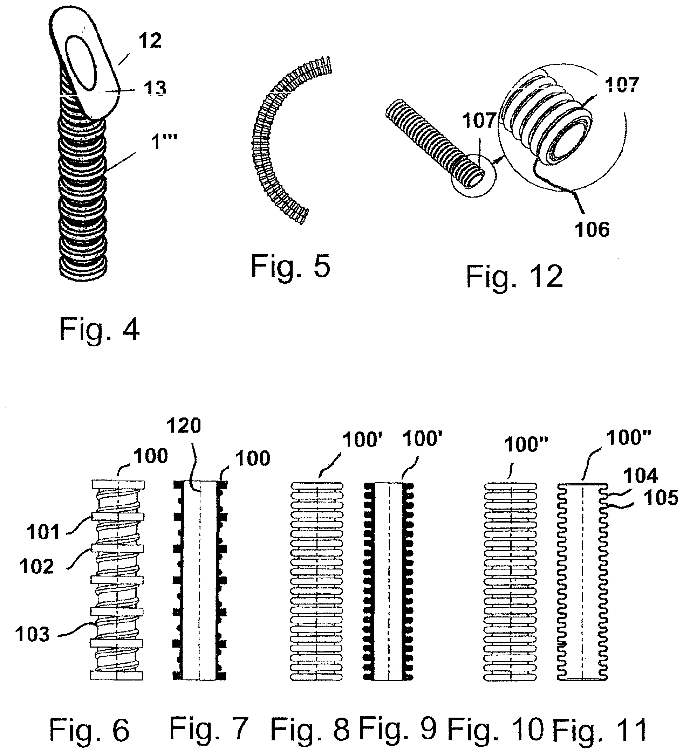

**Figur 4** zeigt eine erfindungsgemäße Kanüle 1''' in perspektivischer Ansicht, die mit äquidistanten Wandverdickungen an ihrer Außenseite entlang ihrer Länge versehen ist. Die Verdickungen sorgen dafür, dass der kreisförmige Querschnitt der Kanüle bei einer Biegung erhalten bleibt. Die verdünnten Bereiche zwischen den Verdickungen erlauben ein einfaches Krümmen der Kanüle. Am herz- oder blutgefäßseitigen Ende ist die Kanüle mit einem Anschlussstück 12 fest verbunden, das einen Anschlussflansch 13, der beispielsweise als Nahtring ausgebildet sein kann, aufweist. Ebenso wie die Kanüle kann auch das Anschlussstück 12 aus Silikon bestehen und mit der Kanüle einstückig hergestellt sein. Ein Flansch 13 kann aus einem filzartigen Gewebe bestehen und mit dem Anschlussstück 12 durch eine übliche Fügetechnik wie Kleben oder auch Nähen verbunden sein. Der Nahtring ist dann andererseits mit einem Körpergewebe durch Nähen verbindbar. Der Nahtring kann bereits eine negativ zylindrische Verkrümmung aufweisen. Es kann auch ein anderes passendes Anschlussstück vorgesehen sein, beispielsweise eine flache Kreisringscheibe, auch mit einem in das Blutgefäß oder ein Herz reichenden Rohrstück.

**Figur 5** zeigt schematisch, dass die erfindungsgemäße Kanüle auch im entspannten Zustand vorgebogen sein kann. Die Abbildung zeigt eine einzige Krümmung, es kann jedoch auch eine Kombination von zwei aufeinanderfolgenden unterschiedlichen Krümmungen, beispielsweise auch gegensinnigen Krümmungen in Form eines S, vorgesehen sein.

**Figur 6** zeigt in einer Seitenansicht das äußere Profil einer erfindungsgemäßen Kanüle 100, die erste Wandverdickungen 101, 102 aufweist, die äquidistant entlang der Länge der Kanüle verteilt und ringförmig azimutal umlaufend sind. Zusätzlich ist eine spiralig umlaufende Verdickung 103 gezeigt. Es können auch nur die Verdickungen 101, 102 oder nur die spiralig umlaufende Verdickung 103 vorgesehen sein.

Die Verdickungen sind, wie in **Figur 7** im Längsschnitt dargestellt, jeweils nur auf der Außenseite der Kanüle 100 vorgesehen, so dass der Innendurchmesser entlang der Kanüle, zumindest zwischen den Endbereichen der Kanüle, konstant bleibt.

In **Figur 8** ist eine Variante der Kanüle dargestellt, in der ausschließlich gleichartig geformte und äquidistant zueinander an der Außenseite der Kanüle 100' angeordnete ringförmige Verdickungen dargestellt sind, wobei die Verdickungen im Längsschnitt der Kanüle eine runde oder, genauer, abgerundete Kontur aufweisen. Die Verdickungen der Kanüle 100 aus Fig. 6 weisen dagegen im Längsschnitt eine rechteckige Kontur auf.

Gemäß **Figur 9****,** die die Variante aus Fig. 8 in einem Längsschnitt zeigt, weist die Kanüle 100' entlang ihrer Länge wie die Kanüle 100 einen konstanten Innendurchmesser auf. Hierdurch wird die Strömung durch das Innere der Kanüle möglichst wenig behindert oder gebremst.

**Figur 10** zeigt in einer Außenansicht eine Kanüle 100'', die als Wellschlauch ausgeführt ist und äquidistante Durchmessererweiterungen entlang ihrer Länge aufweist. Die Wanddicke der Kanüle 100'' ist dabei entlang der Kanülenachse gleichbleibend, so dass jede Erweiterung und Vergrößerung des Außendurchmessers auch eine entsprechende Vergrößerung des Innendurchmessers an derselben Stelle mit sich bringt **(****Figur 11****).** Ebenso verhält es sich mit den Durchmesserverringerungen. Auf diese Weise entsteht eine entlang der Kanülenachse gewellte Außenoberfläche, die mit einer ebenso gewellten Innenoberfläche einhergeht. Auch durch eine solche Struktur wird eine leichte Biegsamkeit der Kanüle 100'' ermöglicht, wobei der Durchmesser, insbesondere der Innendurchmesser, der Kanüle auch für den Fall einer Krümmung stabilisiert ist.

**Figur 12** zeigt exemplarisch zwei Maxima 104, 105 des Außendurchmessers der Kanüle 100'', die von einem Minimum des Außendurchmessers voneinander getrennt sind.

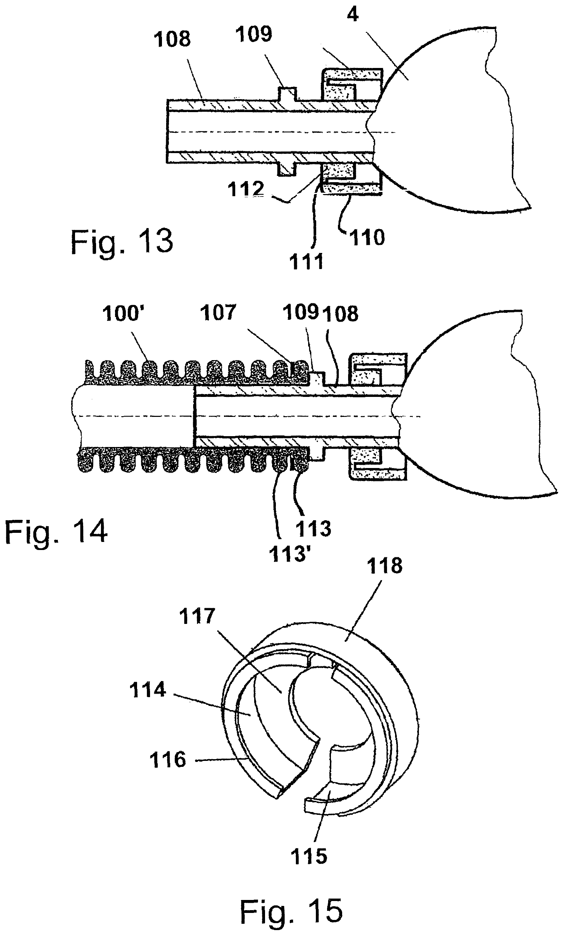

Ein solches Minimum des Außendurchmessers ist in Fig. 12 mit 106 bezeichnet. Solche Minima des Außendurchmessers der Kanüle können entweder durch aufeinanderfolgende Wandverdickungen in dem zwischen den Verdickungen liegenden Bereich oder bei einem Wellschlauch zwischen den Maxima des Außendurchmessers positioniert sein. In Fig. 12 ist dargestellt, dass in dem Bereich 106 des verringerten Außendurchmessers ein Spannring 107 angeordnet ist, der beispielsweise aus Metall, z.B. aus Titan, bestehen kann. Dieser bietet eine gute Anlagefläche für einen in den Figuren 15 bis 18 gezeigten Profilring zum axialen Zusammenhalten einer Verbindung mit einem Stutzen. Er drück gegebenenfalls auch die Kanüle auf den in ihn eingeschobenen, in Fig. 12 nicht dargestellten Stutzen auf.

**Figur 13** zeigt einen Stutzen 108 eines Kanülenverbinders, der im Wesentlichen die Form eines Rohres aufweist und aus Titan bestehen kann. Der Stutzen kann als sogenannte Olive ausgeführt und mit einer sägezahnartigen Außenstruktur zur besseren Herstellung einer Dichtung versehen sein. Der Stutzen 108 weist auf seiner Außenseite einen azimutal umlaufenden Steg 109 mit rechteckigem Querschnitt auf. Auf den Stutzen 108 kann eine Kanüle bis zu dem Steg 109 aufgeschoben und dort fixiert werden.

Der Stutzen 108 ist zudem mit einer Pumpe 4 direkt verbunden. Am pumpenseitigen Ende des Kanülenverbinders ist ein umbördelbarer Schnappring 110 vorgesehen, der mittels eines Filmgelenks 111 mit einem Basisring 112 verbunden ist. Der Basisring 112 ist mit dem Stutzen 108 durch Kleben oder Aufklemmen fest verbunden.

Der Stutzen kann stattdessen auch mit einer Fortsetzungskanüle verbunden sein. Der Schnappring kann dann z.B. mit der Fortsetzungskanüle einstückig zusammenhängen. Die Verbindung kann mit einer Fadenwicklung gesichert sein.

In **Figur 14** ist genauer dargestellt, dass eine Kanüle 100' auf den Stutzen 108 bis zu dem Steg 109 aufgeschoben ist. In die Vertiefung zwischen den ersten beiden Wandverdickungen 113, 113' ist ein Spannring 107 eingelegt, der als Lager für einen Profilring dienen kann. Der Profilring kann allerdings auch direkt axial gegen ein Wanddickenmaximum 113 drücken.

**Figur 15** zeigt in dreidimensionaler Ansicht einen Profilring, der aus zwei Ringsektoren 114, 115 besteht. Die Ringsektoren 114, 115 stellen jeweils einen halbkreisförmigen Teilring dar. Jeder der Ringsektoren 114, 115 weist jeweils zwei radial nach innen ragende Schenkel 116, 117 auf.

Die Profilringsektoren 114, 115 sind durch einen Haltering 118 insgesamt radial zusammengehalten. Der Haltering 118 ist längs geschlitzt, so dass er radial insgesamt aufweitbar oder aufklappbar ist. Die Ringsektoren 114, 115 und der Haltering 118 können aus einem federnden Metall oder einem elastischen Kunststoff bestehen.

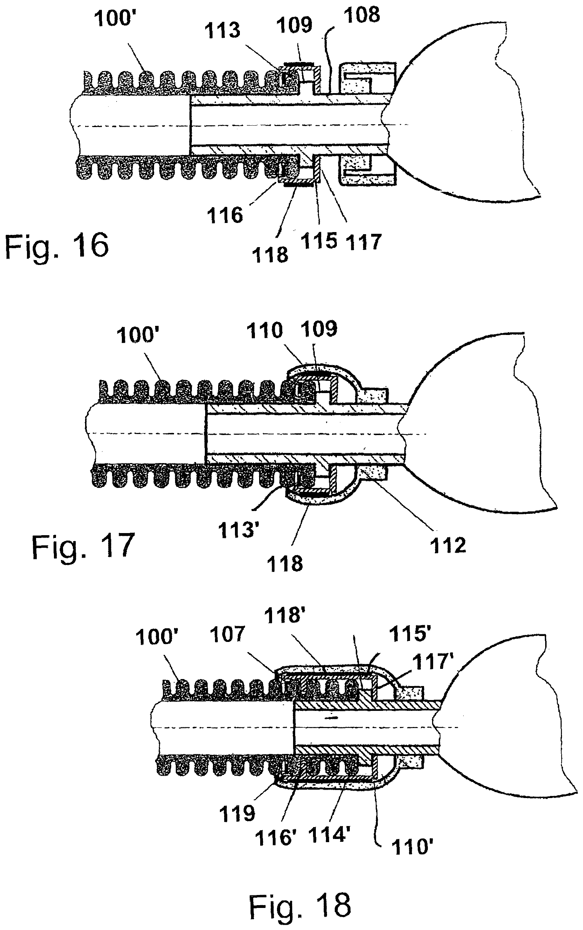

**Figur 16** zeigt, dass die Profilringteile 114, 115 zum axialen Zusammenhalten der Kanüle 100' und des Kanülenverbinders, insbesondere des Stutzens 108, eingesetzt sind. Es ist gezeigt, dass ein Profilring 114, 115 mit seinem einen, längeren Schenkel 117 an der der Kanüle 100' abgewandten Seite des am Stutzen umlaufenden Steges 109 anliegt, während der kürzere Schenkel 116 der Ringsektoren hinter der ersten Verdickung 113 der Kanüle 100' eingreift. Ein Spannring 107 an dieser Stelle kann der besseren Kraftübertragung und einem besseren Halt des Profilrings dienen. Dadurch wird die erste Verdickung 113 axial gegen den Steg 109 gedrückt oder zumindest in Axialrichtung gehalten, und Kanüle und Stutzen werden zusammengehalten. Die beiden Hälften 114, 115 des Profilrings werden durch einen außen aufgesetzten Haltering 118 und/oder durch den Schnappring 110, 110' (Figuren 17 und 18) radial zusammengehalten.

**Figur 17** zeigt im nächsten Schritt in der Weiterentwicklung der Situation aus Fig. 16 eine entsprechende Konstellation, wobei nach dem Aufbringen des Halterings 118 zusätzlich noch der Schnappring 110 über den Profilring und den Haltering bis zur zweiten Verdickung 113' der Kanüle 100' aufgeschnappt ist. Dadurch wird der Übergangsbereich zwischen der Kanüle 100' und dem Steg 109 abgedichtet, und die Profilringteile werden in ihrer Position gesichert.

**Figur 18** zeigt eine Abwandlung des Kanülenverbinders, bei der die Sektoren des Profilrings 114', 115' im Querschnitt einen ersten längeren U-Schenkel 117' und einen zweiten, kürzeren U-Schenkel 116' aufweisen, wobei die Basis der U-Schenkel kanülenseitig über den kürzeren Schenkel 116' in einem freien Ende 119 hinausragt. Dadurch wird in diesem Bereich ein weiterer Teil der Kanüle 100' überdeckt, so dass beispielsweise auch der Spannring 107 in einen Zwischenraum zwischen zwei Verdickungen der Wand der Kanüle 100' positioniert werden kann, der nicht mit demjenigen Zwischenraum identisch ist, in den der kürzere Schenkel 116' eingreift. Der Zwischenraum, in dem der Spannring 107 positioniert ist, ist somit einerseits von dem Profilring, gegebenenfalls auch von dem Haltering 118' und zudem von dem Schnappring 110' überdeckt, der ebenfalls länger ausgebildet sein kann als der Schnappring 110.

Durch eine lange Überdeckung des Stutzens 108 mit der Kanüle kann eine flüssigkeitsdichte Verbindung dort auch dann garantiert werden, wenn das Ende der Kanüle möglicherweise beschädigt oder ungünstig gestaltet ist, beispielsweise durch Einschnitte oder schräges Abschneiden. Durch die Gestaltung des Kanülenverbinders wird es auch unter schwierigen Operationsbedingungen einfach möglich, die Kanüle flüssigkeitsdicht mit einem Pumpenanschluss zu verbinden.

## Patentansprüche

1. Kanüle (1; 1'; 1"; 100; 100'; 100") in Form eines zur Bildung eines Fließkanals für Körperflüssigkeiten, insbesondere für Blut, ausgebildeten Hohlkörpers mit einem ersten Ende (2), das zur mittelbaren oder unmittelbaren Verbindung mit einem Körperorgan oder einem Körperflüssigkeit führenden, insbesondere körpereigenen Hohlraum ausgebildet ist, und mit einem zweiten Ende (3), das zur Verbindung mit einem Leitungselement oder einem Funktionselement (4) der Flüssigkeitsförderung ausgebildet ist, wobei die Kanüle wenigstens zwischen ihren Enden aus einem einzigen, homogenen, flüssigkeitsdichten Material besteht, das elastisch und/oder plastisch verformbar ist, wobei die Kanüle durch Spritzgießen aus Silikon, einem Silikonelastomer oder einem Polyurethan, hergestellt ist und wobei die Wanddicke und/oder der Durchmesser der Kanüle im Längsschnitt zwischen dem ersten und dem zweiten Ende im Profil zwei oder mehr Maxima und Minima aufweist.

2. Kanüle nach Anspruch 1, **dadurch gekennzeichnet, dass** im Längsschnitt wiederkehrende Wandverdickungen (101, 102) vorgesehen sind, die jeweils kreisringförmig umlaufend ausgebildet sind.

3. Kanüle nach Anspruch 1, **dadurch gekennzeichnet, dass** längs der Kanüle eine oder mehrere wendelförmig umlaufende Wandverdickungen (103) vorgesehen sind.

4. Kanüle nach einem der Ansprüche 1 bis 3, **dadurch gekennzeichnet, dass** der Innendurchmesser der Kanüle im Bereich von Verdickungen (101, 102, 103) der Kanülenwand entlang der Längsachse (120) der Kanüle konstant ist.

5. Kanüle nach einem der Ansprüche 1 bis 4, **dadurch gekennzeichnet, dass** die Kanüle an ihrem ersten Ende (2) mit einem Anschlussstück (5, 12, 13), insbesondere einem Anschlussflansch, zur Verbindung mit einem Körpergewebe versehen ist.

6. Kanüle nach Anspruch 5, **dadurch gekennzeichnet, dass** das Anschlussstück (5, 12, 13) zusammen mit den übrigen Teilen der Kanüle einstückig durch Spritzgießen hergestellt ist.

7. Kanüle nach einem der vorangehenden Ansprüche, **dadurch gekennzeichnet, dass** die Kanüle aus Silikon besteht.

8. Kanüle nach einem der vorangehenden Ansprüche, **dadurch gekennzeichnet, dass** das Wandprofil der Kanüle im Längsschnitt entlang ihrer Längsachse wiederkehrende, insbesondere äquidistante Durchmesserveränderungen und/oder Wandverdickungen (101, 102, 103) aufweist.

9. Kanüle nach Anspruch 1 oder einem der folgenden, **dadurch gekennzeichnet, dass** die Kanüle im entspannten Zustand eine Vorzugskrümmung aufweist.

10. Kanülenverbinder für eine Kanüle gemäß einem der Ansprüche 1 bis 9, **gekennzeichnet durch** einen in die Kanüle einsteckbaren Stutzen (108), einen außen an dem Stutzen azimutal umlaufenden Steg (109) sowie einen mehrteiligen Profilring, der ausgestaltet ist, den Steg und wenigstens einen ersten bezüglich des Außendurchmessers ein Maximum aufweisenden Längenbereich der Kanüle zu umgreifen, wobei der Profilring zwei oder mehr als zwei voneinander getrennte Ringsektoren (114, 115) aufweist.

11. Kanülenverbinder nach Anspruch 10, **dadurch gekennzeichnet, dass** wenigstens einer der Ringsektoren im Querschnitt wenigstens einen, insbesondere zwei radial nach innen ragende Schenkel (116, 117) aufweist und wobei insbesondere ein umbördelbarer, die Ringsektoren im eingeschnappten Zustand radial haltender Schnappring (110, 110') vorgesehen ist.

12. Verfahren zum Herstellen einer Kanüle nach einem der Ansprüche 1 bis 11, **dadurch gekennzeichnet, dass** die Kanüle durch Spritzgießen hergestellt wird.

13. Verfahren nach Anspruch 12, **dadurch gekennzeichnet, dass** die Kanüle aus Silikon hergestellt wird.

14. Verfahren nach Anspruch 12 oder 13, soweit dieser Anspruch auf Anspruch 5 oder 6 zurückbezogen ist, **dadurch gekennzeichnet, dass** des Anschlussstück (5, 12, 13) zusammen mit den übrigen Teilen der Kanüle einstückig durch Spritzgießen hergestellt wird.

## Claims

1. A cannula (1; 1'; 1"; 100; 100'; 100") in the form of a hollow body embodied for forming a flow channel for bodily fluids, in particular for blood and having a first end (2) that is embodied for indirect or direct connection to a bodily organ or to a hollow chamber, in particular a bodily hollow chamber, that conducts a bodily fluid, and having a second end (3) that is embodied for connecting to a line element or a functional element (4) for conveying fluid, wherein the cannula, at least between its ends, comprises a single, homogeneous, fluid-tight material that is elastically and/or plastically deformable, wherein the cannula is produced from silicone, a silicone elastomer, or a polyurethane, using injection molding, and wherein the wall thickness and/or the diameter of the cannula in the longitudinal section between the first and the second end in the profile has two or more maxima and minima.

2. The cannula according to claim 1, **characterized in that** recurring wall thicknesses (101, 102) are provided in the longitudinal section that are each embodied circumferentially in a circular ring-shaped manner.

3. The cannula according to claim 1, **characterized in that** one or a plurality of helical circumferential wall thickenings (103) are provided along the cannula.

4. The cannula according to any of claims 1 through 3, **characterized in that** the inner diameter of the cannula in the region of thickenings (101, 102, 103) in the cannula wall is constant along the longitudinal axis (120) of the cannula.

5. The cannula according to any of claims 1 through 4, **characterized in that** at its first end (2) the cannula is provided with a connecting piece (5, 12, 13), in particular a connecting flange, for connecting to a body tissue.

6. The cannula according to claim 5, **characterized in that** the connecting piece (5, 12, 13), together with the other parts of the cannula is produced in a single piece using injection molding.

7. The cannula according to any of the previous claims, **characterized in that** the cannula consists of silicone.

8. The cannula according to any of the previous claims, **characterized in that** the wall profile of the cannula in the longitudinal section has recurring changes in diameter and/or wall thickenings (101, 102, 103), in particular equidistant changes in diameter and/or wall thickenings, along its longitudinal axis.

9. The cannula according to claim 1 or any of the following claims, **characterized in that** in the relaxed state the cannula has a spontaneous curvature.

10. A cannula connector for a cannula according to any of claims 1 through 9, **characterized in that** by a connecting piece (108) that may be inserted into the cannula, a bar (109) exteriorly azimuthally surrounding the connecting piece, and a multi-part profile ring that is embodied to grip the bar and at least a first longitudinal region of the cannula having a maximum with respect to the outer diameter,
wherein the profile ring has two or more than two annular sectors (114, 115) separated from one another.

11. A cannula connector according to claim 10, **characterized in that** at least one of the annular sectors, in section, has at least one, in particular two, radially inwardly projecting legs (116, 117), and wherein in particular a snap-fit ring (110, 110') that may be beaded and that holds the annular sectors radially when snapped in is provided.

12. A method for producing a cannula according to any of claims 1 through 11, **characterized in that** the cannula is produced using injection molding.

13. The method according to claim 12, **characterized in that** the cannula is produced from silicone.

14. The method according to claim 12 or 13, if this claim refers back to claim 5 or 6, **characterized in that** the connecting piece (5, 12, 13) together with the other parts of the cannula are produced in a single piece using injection molding.

## Revendications

1. Canule (1 ; 1' ; 1" ; 100 ; 100' ; 100") sous forme d'un corps creux, constitué pour la formation d'un canal d'écoulement pour des fluides corporels, notamment du sang, doté d'une première extrémité (2), qui est conçue pour la liaison directe ou indirecte avec un organe corporel ou un corps creux emmenant un fluide corporel, notamment propre au corps, et doté d'une seconde extrémité (3), qui est conçue pour la liaison avec un élément de conduite ou un élément fonctionnel (4) du transport de fluides, où la canule est constituée au moins entre ses extrémités d'un matériau unique, homogène, étanche aux fluides, qui est déformable de manière élastique et/ou plastique, où la canule est fabriquée par un moulage par injection d'un silicone, d'un élastomère de silicone ou d'un polyuréthane, et où l'épaisseur de paroi et/ou le diamètre de la canule en coupe longitudinale présente entre la première et la seconde extrémité deux ou plus de deux maximums et minimums dans le profil.

2. Canule selon la revendication 1, **caractérisée en ce qu'**en coupe longitudinale, des surépaisseurs de paroi (101, 102) récurrentes sont prévues, qui sont conçues respectivement circonférentielles en forme de cercles.

3. Canule selon la revendication 1, **caractérisée en ce que** le long de la canule une ou plusieurs surépaisseurs de paroi (103) circonférentielles en forme d'hélices sont prévues.

4. Canule selon l'une des revendications 1 à 3, **caractérisée en ce que** le diamètre intérieur de la canule est constant dans la zone des surépaisseurs (101, 102, 103) de la paroi de canule le long de l'axe longitudinal (120) de la canule.

5. Canule selon l'une des revendications 1 à 4, **caractérisée en ce que** la canule est munie d'une pièce de raccordement (5, 12, 13), notamment d'une bride de raccordement, à sa première extrémité (2), pour la liaison avec un tissu corporel.

6. Canule selon la revendication 5, **caractérisée en ce que** la pièce de raccordement (5, 12, 13) est fabriquée d'un seul tenant par un moulage par injection conjointement avec les autres pièces de la canule.

7. Canule selon l'une des revendications précédentes, **caractérisée en ce que** la canule est constituée de silicone.

8. Canule selon l'une des revendications précédentes, **caractérisée en ce que** le profil de paroi de la canule en coupe longitudinale présente des variations de diamètres et/ou des surépaisseurs de paroi (101, 102, 103) récurrentes, notamment équidistantes, le long de son axe longitudinal.

9. Canule selon la revendication 1 ou l'une des suivantes, **caractérisée en ce que** la canule présente une courbure préférentielle à l'état déployé.

10. Connecteur de canule pour une canule selon l'une des revendications 1 à 9, **caractérisé par** un embout (108) pouvant être inséré dans la canule, une tige (109) entourant par l'extérieur de manière azimutale l'embout, ainsi qu'un anneau de profil en plusieurs parties qui est conçu pour prendre en prise en l'entourant la tige et au moins une première région en longueur de la canule par rapport au diamètre extérieur présentant un maximum,
où l'anneau de profil présente deux ou plus de deux secteurs annulaires (114, 115) séparés l'un de l'autre.

11. Connecteur de canule selon la revendication 10, **caractérisé en ce qu'**au moins un des secteurs annulaires présente dans la section transversale au moins un, notamment deux épaulements (116, 117) allant radialement vers l'intérieur, et où, en particulier, est prévu un circlip (110, 110') pouvant être rabattu sur son pourtour, maintenant radialement les secteurs annulaires dans un état d'emprise.

12. Procédé de fabrication d'une canule selon l'une des revendications 1 à 11, **caractérisé en ce que** la canule est fabriquée par un moulage par injection.

13. Procédé selon la revendication 12, **caractérisé en ce que** la canule est fabriquée en silicone.

14. Procédé selon la revendication 12 ou 13, dans la mesure où cette revendication dépend de la revendication 5 ou 6, **caractérisé en ce que** la pièce de raccord (5, 12, 13) est fabriquée d'un seul tenant conjointement avec les autres pièces de la canule par un moulage sou pression.
